# EUROPEAN PATENT APPLICATION

(11) **EP 3 058 862 A1**
(43) Date of publication of application: **24.08.2016**
(21) Application number: 14853805.1
(22) Date of filing: 15.10.2014
(51) Int. Cl.: A61B 1/00, A61B 90/00

(54) **MEDICAL DEVICE**

(30) Priority: 15.10.2013 US 201361891137 P
(71) Applicant: OLYMPUS CORPORATION, Hachioji-shi, Tokyo 192-8507 (JP)
(72) Inventor: MIKKAICHI, Takayasu, Tokyo 151-0072 (JP); AIKAWA, Yoshie, Tokyo 151-0072 (JP)
(74) Representative: Schicker, Silvia
(86) International application number: PCT/JP2014/077394
(87) International publication number: WO 2015/056693

(57) **Abstract**

A medical device includes: an insertion portion that includes a distal end and a proximal end and is capable of being inserted into a body; a light-emitting portion that is disposed at the distal end of the insertion portion and is configured to emit light containing a wavelength capable of passing through a biological tissue; and a marking portion that is disposed at the distal end of the insertion portion and is configured to form a marking on the biological tissue. The light-emitting portion and the marking portion are disposed at the insertion portion such that a positional relationship between the light-emitting portion and the marking portion can be fixed.

## Description

### Technical Field

The present invention relates to a medical device.

Priority is claimed on US Provisional Patent Application No. 61/891,137, filed October 15,2013, the content of which is incorporated herein by reference.

### Background Art

In the related art, in order to accurately excise an excision target site in a widely-used surgical operation by which a lesion site on the mucous membrane of a hollow organ is excised, an operator confirms the lesion site from the inside of the hollow organ, forms a marking at an excising position, and incises the site at a marking position from an abdominal cavity side. Marking using an ink dot, marking by indwelling a clip, marking by cauterizing, and the like are known as methods of forming a marking on an excision target site from the inside of a hollow organ. For example, PTL 1 discloses a living body observation apparatus including means for forming a marking on a target using a dyeing colorant. PTL 1 discloses a technique in which a marking is formed by transmuting the surface of a target using heat or high-frequency waves instead of using a dyeing colorant.

### Citation List

### Patent Literature

PTL 1: Japanese Unexamined Patent Application, First Publication No. 2009-153828

### Summary of Invention

### Technical Problem

In the related art, when an operator forms markings on surfaces opposed to each other such as an internal surface and an external surface of a stomach, the operator forms one of the markings on one surface, and then shows the position of the marking on an opposite surface side by pressing the marked site to cause protrusion on the opposite surface. In another case, the operator forms the marking on the one surface, and then shows the position of the marking on the opposite surface side by replacing the marking formation instrument with a known instrument with a light-emitting distal end, and bringing light into contact with the position of the marking.

However, it is difficult to accurately show the position of the marking to the opposite surface by the method in which the marked site is pressed to protrude the corresponding site on the opposite surface. As a result, it is necessary to widely excise a lesion site to prevent the occurrence of the remainder of the lesion site, and the scale of invasion may be increased. According to the method in which the marking formation instrument is replaced with the light-emitting instrument and the position of a marking is shown using light, the replacement of the instruments takes time and effort, and it is necessary to accurately bring light into contact with the position of the marking, thereby requiring a complicated technique.

The present invention is made in light of this problem, and an object of the present invention is to provide a medical device capable of accurately forming markings both of an internal surface and an external surface of a biological tissue within a short period of time.

### Solution to Problem

According to a first aspect of the present invention, a medical device includes: an insertion portion that includes a distal end and a proximal end and that is capable of being inserted into a body; a light-emitting portion that is disposed at the distal end of the insertion portion and that is configured to emit light containing a wavelength capable of passing through a biological tissue; and a marking portion that is disposed at the distal end of the insertion portion, that has a fixed positional relationship with the light-emitting portion, and that is configured to form a marking on the biological tissue.

According to a second aspect of the present invention, in the medical device according to the first aspect, the insertion portion may include a longitudinal axis that connects the distal end and the proximal end together, and in a cross-sectional surface, which is perpendicular to the longitudinal axis, of the distal end of the insertion portion, the light-emitting portion may be disposed in an inner region of a contour of the marking portion or in an inner region of an envelope of a portion of the marking portion which is capable of coming into contact with the biological tissue.

According to a third aspect of the present invention, in the medical device according to the first aspect, the marking portion and the light-emitting portion may be capable of being coaxially positioned.

According to a fourth aspect of the present invention, in the medical device according to the first aspect, the marking portion may include an electrode capable of being energized with high-frequency current to cauterize the biological tissue.

According to a fifth aspect of the present invention, in the medical device according to the first aspect, the light-emitting portion may be capable of emitting light, which has energy required to cauterize the biological tissue, to the biological tissue, and a change in the energy may allow the light-emitting portion to serve as the marking portion.

### Advantageous Effects of Invention

According to these aspects, it is possible to accurately form markings on both an internal surface and an external surface of a biological tissue within a short period of time.

### Brief Description of Drawings

FIG. 1 is an overall view illustrating a medical device according to a first embodiment of the present invention.
FIG. 2 is a front view of the medical device according to the first embodiment of the present invention.
FIG. 3 is a sectional view of a distal portion of the medical device according to the first embodiment of the present invention.
FIG. 4 is a schematic view illustrating the configuration of the medical device according to the first embodiment of the present invention.
FIG. 5 is a view illustrating a method of using the medical device according to the first embodiment of the present invention.
FIG. 6 is a view illustrating the method of using the medical device according to the first embodiment of the present invention.
FIG. 7 is a view illustrating the method of using the medical device according to the first embodiment of the present invention.
FIG. 8 is a view illustrating the method of using the medical device according to the first embodiment of the present invention.
FIG. 9 is a view illustrating a state after marking is performed using the medical device according to the first embodiment of the present invention.
FIG. 10A is a sectional view of a distal portion of a medical device according to a second embodiment of the present invention for illustrating the medical device.
FIG. 10B is a sectional view illustrating the configuration of a modified example of the second embodiment of the present invention.
FIG. 11 is a partial sectional view of a medical device according to a third embodiment of the present invention for illustrating the medical device.
FIG. 12 is a front view of the medical device according to the third embodiment of the present invention.
FIG. 13 is a view illustrating a state after marking is performed using the medical device according to the third embodiment of the present invention.
FIG. 14 is a view illustrating a positional relationship between markings formed by the medical device according to the third embodiment of the present invention.
FIG. 15 is a partial sectional view of a medical device according to a fourth embodiment of the present invention for illustrating the medical device.
FIG. 16 is an overall view illustrating a medical device according to a fifth embodiment of the present invention.
FIG. 17 is an overall view illustrating a medical device according to a sixth embodiment of the present invention.
FIG. 18 is a schematic view illustrating the configuration of the medical device according to the sixth embodiment of the present invention.

### Description of Embodiments

### (First Embodiment)

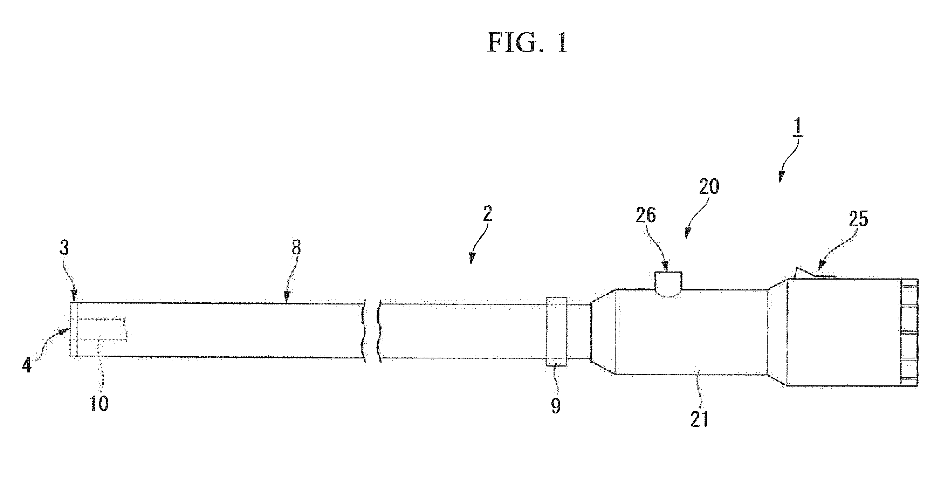

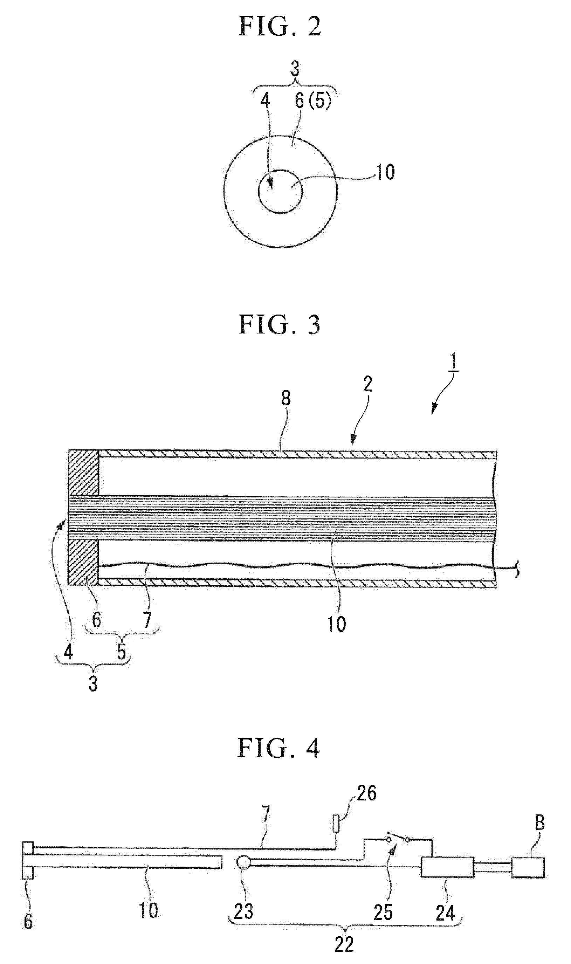

A medical device 1 according to a first embodiment of the present invention will be described. FIG. 1 is an overall view illustrating the medical device 1. FIG. 2 is a front view of the medical device 1. FIG. 3 is a sectional view of a distal portion of the medical device 1. FIG. 4 is a schematic view illustrating the configuration of the medical device 1.

As illustrated in FIG. 1, the medical device 1 includes an insertion portion 2 having a distal end and a proximal end, and a body portion 20 provided at the proximal end of the insertion portion 2.

As illustrated in FIGS. 1 and 2, the insertion portion 2 is a flexible long member inserted into a human body. The insertion portion 2 includes a distal configuration portion 3, a tubular portion 8, and a light guide 10.

The distal configuration portion 3 is provided in a distal portion of the insertion portion 2. The distal configuration portion 3 includes a light-emitting portion 4 and a marking portion 5.

The light-emitting portion 4 is disposed at a distal end of the light guide 10. Light transmitted via the light guide 10 to the distal end of the light guide 10 is emitted from the light-emitting portion 4. The light-emitting portion 4 is disposed in a central portion of the insertion portion 2 in a cross-sectional surface perpendicular to a longitudinal axis of the insertion portion 2 that connects the distal end and the proximal end of the insertion portion 2 together.

As illustrated in FIGS. 2 and 3, the marking portion 5 includes an annular electrode (an electrode) 6 having an annular shape surrounding a circumference of the light-emitting portion 4 in the cross-sectional surface perpendicular to the longitudinal axis of the insertion portion 2, and a wiring 7 connected to the annular electrode 6. In the embodiment, the annular electrode 6 of the marking portion 5 has a circular plate shape having a through-hole at the center thereof. The distal end of the light guide 10 is inserted into the through-hole of the annular electrode 6. That is, in the embodiment, the light-emitting portion 4 is disposed in a region inside of an inner contour of the annular electrode 6 in the cross-sectional surface perpendicular to the longitudinal axis of the insertion portion 2. A lens may be provided in the through-hole of the annular electrode 6 so as to control distribution of light emitted from the distal end of the light guide 10.

In the embodiment, a position of the light-emitting portion 4 and a position of the marking portion 5 have a fixed relationship with each other.

The wiring 7 is a conductor that electrically connects the annular electrode 6 with a connector 26 (to be described later).

The tubular portion 8 is a flexible cylindrical member connected to a proximal end of the distal configuration portion 3. In the embodiment, at least a distal portion of the tubular portion 8 is an elastic member having resilience such that the elastic member is maintained straight when external force is not applied thereto. The light guide 10 and the wiring 7 are inserted into the tubular portion 8. A proximal end of the tubular portion 8 is fixed to the body portion 20. In the embodiment, a stopper 9 that can be engaged with a forceps port provided at a proximal end of a treatment tool channel of a flexible endoscope is mounted on the tubular portion 8.

As illustrated in FIGS. 1 and 4, a proximal end of the light guide 10 is disposed in the body portion 20, and the distal end of the light guide 10 is disposed in the light-emitting portion 4. The light guide 10 is a bundle of optical fibers through which light is transmitted from the body portion 20 to the light-emitting portion 4.

The body portion 20 includes an exterior body 21 to which the proximal end of the tubular portion 8 is fixed, a light source unit 22 disposed inside the exterior body 21, and the connector 26 fixed to the exterior body 21.

The light source unit 22 includes a light emitter 23, a power supply unit 24, and a switch 25.

The positional relationship of the light emitter 23 relative to the light guide 10 is determined such that light is incident to the proximal end of the light guide 10. For example, a light-emitting diode, a laser diode, lamps such as an incandescent lamp, or the like may be appropriately selected and adopted as the light emitter 23. In the embodiment, a light-emitting diode having advantages in terms of the amount of light, heat generation, and power consumption is adopted as the light emitter 23. The wavelength of light emitted from the light emitter 23 is set such that the wavelength of light emitted from the light-emitting portion 4 is from 600 nm to 800 nm. Accordingly, the light emitted from the light-emitting portion 4 becomes a visible light beam suitably passing through a biological tissue. In the embodiment, light emitted from the light-emitting portion 4 preferably contains a small number of wavelength components having wavelengths greater than or equal to those in the infrared region. The reason for this is that in the embodiment, only visible light components of the light emitted from the light-emitting portion 4 are used, and wavelength components in the infrared region are deemed to adversely affect the biological tissue by heating the biological tissue. In the embodiment, light emitted from the light-emitting portion 4 preferably contain almost no wavelength components in the ultraviolet region. In the embodiment, the wavelength of light emitted from the light emitter 23 is set to be from 600 nm to 800 nm. Means for changing the length of the wavelength of the light is not provided on a path from the proximal end of the light guide 10 to the light-emitting portion 4. In the embodiment, output power of the light emitter 23 is less than or equal to 5 mW.

The power supply unit 24 is provided inside the exterior body 21 so as to supply the light emitter 23 with electric power required to emit light by the light emitter 23. In the embodiment, a battery B is provided at the power supply unit 24.

In order for an operator to manually turn on an off the supply of electric power from the power supply unit 24 to the light emitter 23, the switch 25 is provided in the exterior body 21 while being exposed from the external surface of the exterior body 21. A plug cord connected to a well-known high-frequency power supply device can be attached to and detached from the connector 26.

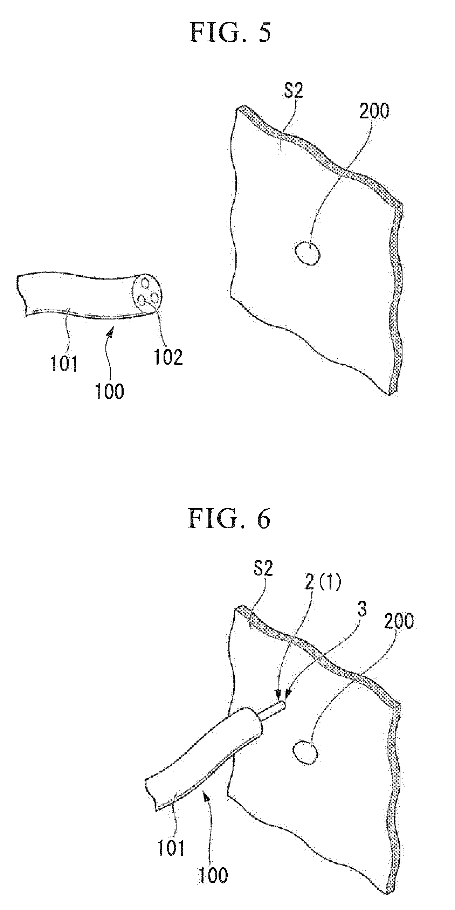

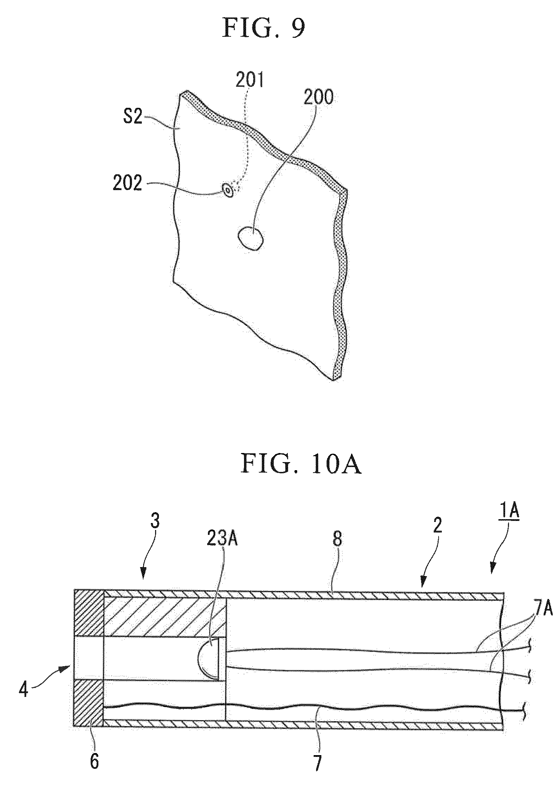

Next, a method of using the medical device 1 according to the embodiment and an operation thereof will be described. FIGS. 5 to 8 are views illustrating the method of using the medical device 1. FIG. 9 is a view illustrating a state after marking is performed using the medical device 1.

As an example of the usage of the medical device 1, an example of forming markings 201 and 202 on the stomach of a patient having a lesion 200 in the stomach so as to indicate the position of the lesion 200 in the stomach is illustrated.

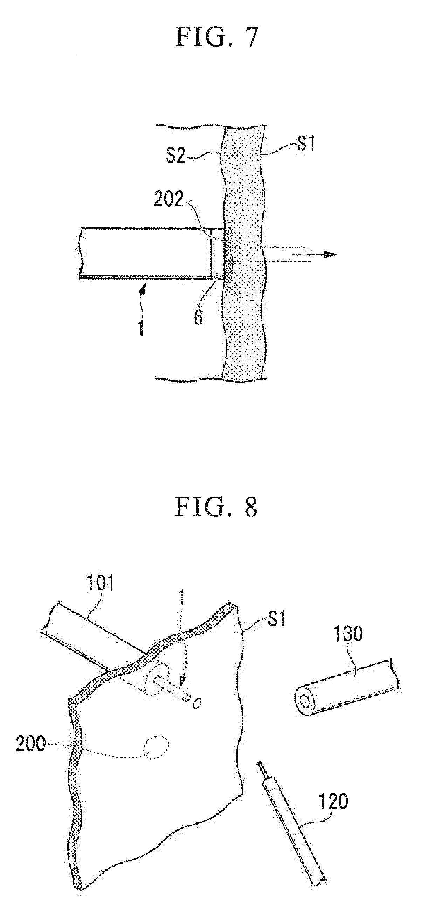

As illustrated in FIG. 5, first, an operator observes the lesion 200 using a well-known flexible endoscope 100. That is, the operator inserts a flexible insertion portion 101 of the well-known flexible endoscope 100 into the stomach through the mouth of the patient, and observes the inside of the stomach. When the operator determines to excise the lesion 200 as a result of observing the lesion 200 in the stomach, the operator incises the abdominal wall, and introduces a well-known instrument 120 used to form the marking 201 on the external surface of the stomach, and a laparoscope 130 (refer to FIG. 8) into the abdominal cavity. As illustrated in FIG. 6, the operator inserts the insertion portion 2 of the medical device 1 according to the embodiment into a treatment tool channel 102 provided in the flexible endoscope 100. In the embodiment, when the medical device 1 is used, the plug cord of the high-frequency power supply device is attached to the connector 26 of the body portion 20 of the medical device 1. A return electrode of the high-frequency power supply device is attached to the body wall of the patient into which the medical device 1 according to the embodiment is inserted.

The insertion portion 2 of the medical device 1 is pushed toward a distal side through the treatment tool channel 102 such that a distal end of the insertion portion 2 of the medical device 1 protrudes from a distal end of the treatment tool channel 102. When the distal end of the insertion portion 2 of the medical device 1 protrudes from the distal end of the treatment tool channel 102, an image of the distal end of the insertion portion 2 is captured in an endoscopic image acquired by the flexible endoscope 100.

As illustrated in FIG. 6, the operator of the medical device 1 and the flexible endoscope 100 guides the distal end of the insertion portion 2 of the medical device 1 to the vicinity of the lesion 200, which is an excision target, by operating the flexible endoscope 100. After guiding the distal end of the insertion portion 2 of the medical device 1 to the vicinity of the lesion 200, the operator brings the distal end of the insertion portion 2 into contact with a site positioned apart by a predetermined distance from the lesion 200. A site on an internal surface S2 of the stomach, with which the distal end of the insertion portion 2 is brought into contact, is positioned apart from the lesion 200 by a margin set depending on the state of the lesion 200.

When the distal end of the insertion portion 2 is brought into contact with the internal surface S2 of the stomach, the annular electrode 6 of the marking portion 5 is brought into contact with the internal surface S2 of the stomach.

The annular electrode 6 is energized with high-frequency current from the high-frequency power supply device via the connector 26 without changing the position of the annular electrode 6 in contact with the internal surface S2 of the stomach. As illustrated in FIG. 7, the contact site on the internal surface S2 of the stomach is cauterized to conform to the shape of the annular electrode 6 so that the annular marking 202 is formed on the internal surface S2 of the stomach, with which the annular electrode 6 is in contact.

Subsequently, the operator turns on the switch 25 of the body portion 20 to allow the light emitter 23 to emit light in a state where the annular electrode 6 is in contact with the internal surface S2 of the stomach. As illustrated in FIG. 8, the light emitted from the light emitter 23 is transmitted to the distal end of the insertion portion 2 through the light guide 10, and is emitted from the light-emitting portion 4. The light emitted from the light-emitting portion 4 passes an external surface (surface on an abdominal cavity side) S1 of the stomach through the stomach wall, and is transmitted to the abdominal cavity. The transmitted light can be observed using an instrument such as a laparoscope inserted into the abdominal cavity. At this time, the light observed in the abdominal cavity indicates a central portion of the annular electrode 6. The light transmitted from the internal surface S2 to the external surface S1 of the stomach is positioned at substantially the center of a site on which the marking 202 is actually formed.

As illustrated in FIG. 8, the operator performs marking using the tool introduced into the abdominal cavity to form the marking 201 on the external surface of the stomach at the position of the light observed via the laparoscope. The marking 201 may be formed using a coloring matter, or via cauterizing using an electric scalpel, a heat probe, or the like.

As illustrated in FIG. 9, the position of the marking 201 on the external surface S1 of the stomach is opposite to the position of the marking 202 on the internal surface S2 of the stomach such that the stomach wall is interposed therebetween.

In the embodiment, the operator may form the marking 201 on the external surface S1 of the stomach prior to forming the marking 202 on the internal surface S2 of the stomach using the annular electrode 6. In this case, the position of the light acquired from the outside of the stomach in a laparoscopic image is coincident with a predetermined forming position at which the marking 202 is formed on the internal surface S2 of the stomach by the medical device 1.

The operator forms the markings 201 on the external surface S1 of the stomach and the markings 202 on the internal surface S2 of the stomach at multiple locations around a circumference of the lesion 200 by the aforementioned same sequence. Thereafter, the operator excises the lesion 200 with reference to the positions of the markings 201 and 202 using a well-known technique.

The medical device 1 according to the embodiment is capable of consecutively or simultaneously performing both the transmission of the predetermined forming position of the marking 202 to the external surface S1 of the stomach, and the formation of a marking on the internal surface S2 of the stomach in a state where the annular electrode 6 of the marking portion 5 is in contact with the internal surface S2 of the stomach.

For this reason, the operator can accurately form markings on both the internal surface S2 and the external surface S1 of the stomach within a short period of time compared to a method in the related art.

Since the annular electrode 6 has an annular shape and the light-emitting portion 4 is disposed in the central portion of the annular electrode 6, even if light is dispersed by the stomach wall, light emitted from the light-emitting portion 4 and observed on the external surface S1 of the stomach is positioned at substantially the center of the annular electrode 6. For this reason, the operator observes the external surface S1 of the stomach and forms a marking at the position of light so that the position of the marking on the external surface S1 of the stomach is easily placed in a marking region formed in an annular shape on the internal surface S2 of the stomach.

In the embodiment, an example, in which the stomach has a lesion to be excised, is described; however, an organ having a lesion is not limited to the stomach. The medical device 1 according to the embodiment can be used in a case where markings are formed on a pair of opposite surfaces of a hollow tissue or other tissues into which an endoscope can be inserted.

The light guide 10 may be advanced and retracted relative to the annular electrode 6. For example, the light guide 10 may be configured such that the light guide 10 can be pulled into the tubular portion 8 when cauterizing is performed using the annular electrode 6. When the light guide 10 can be pulled into the tubular portion 8, thermal damage to the light guide 10 can be prevented.

The light-emitting portion 4 may be advanced and retracted relative to the annular electrode 6. For example, the light-emitting portion 4 may be configured such that the light-emitting portion 4 can pulled into the tubular portion 8 when cauterizing is performed using the annular electrode 6. When the light-emitting portion 4 can be pulled into the tubular portion 8, dirt generated during the cauterizing of a tissue is unlikely to get stuck to the light-emitting portion 4.

### (Second Embodiment)

A second embodiment of the present invention will be described. In each embodiment to be described below, the same reference signs are assigned to elements having the same configurations as those of the medical device 1 described in the first embodiment, and duplicate description of the first embodiment will be omitted. FIG. 10A is a sectional view of a distal portion of a medical device 1A according to the embodiment for illustrating the medical device 1A.

As illustrated in FIG. 10A, the medical device 1A according to the embodiment has a configuration different from the medical device 1 described in the first embodiment in that the light guide 10 is not provided and a light emitter 23A is provided in a distal portion of the insertion portion 2 instead of providing the light emitter 23 in the body portion 20.

The light emitter 23A is fixed to the distal configuration portion 3 such that light can be emitted from the light-emitting portion 4. A wiring 7A connected to the switch 25 and the power supply unit 24 is attached to the light emitter 23A. Accordingly, the light emitter 23A can be turned on and off by the operating of the switch 25.

Similar to the first embodiment, a position of the light-emitting portion 4 and a position of the marking portion 5 have a fixed relationship with each other in the medical device 1A according to the embodiment. The medical device 1A according to the embodiment achieves the same effects as in the first embodiment.

Part of light emitted from the light emitter 23 of the medical device 1 according to the first embodiment that uses the light guide 10 is attenuated until the emitted light reaches the light-emitting portion 4. In contrast, in the embodiment, since the light emitter 23A is provided in the distal portion of the insertion portion 2, the amount of light attenuation is small compared to when the light guide 10 is used. When the light emitter 23A is provided in the distal portion of the insertion portion 2, the total extension length of the wiring 7A for supplying electric power required to cause the light emitter 23A to emit light may be large compared to that in the first embodiment. However, when the attenuation of light energy induced by the light guide 10 is compared to an electric power loss induced by the wiring 7A, even if the occurrence of an electric energy loss is caused by the wiring 7, an energy loss when the light emitter 23A is provided in the distal portion of the insertion portion 2 is small compared to that induced by the light guide 10.

### (Modified example)

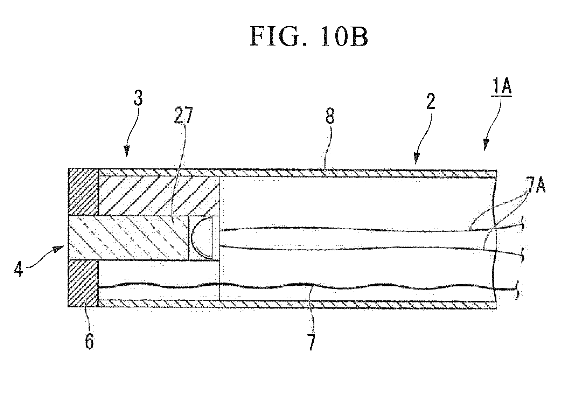

Next, a modified example of the embodiment will be described. FIG. 10B is a sectional view illustrating the configuration of the modified example.

In the modified example, a rod 27 having light transmittance is provided on a distal side of the light emitter 23A. For example, the rod 27 is made of glass, and has durability against heat generated when the annular electrode 6 is energized with high-frequency current. A distal end surface of the rod 27 is formed to have a proper shape, thereby allowing the controlling of the distribution of light emitted from a distal end of the rod 27.

Also, the same effects as in the embodiment are obtained in this configuration.

### (Third Embodiment)

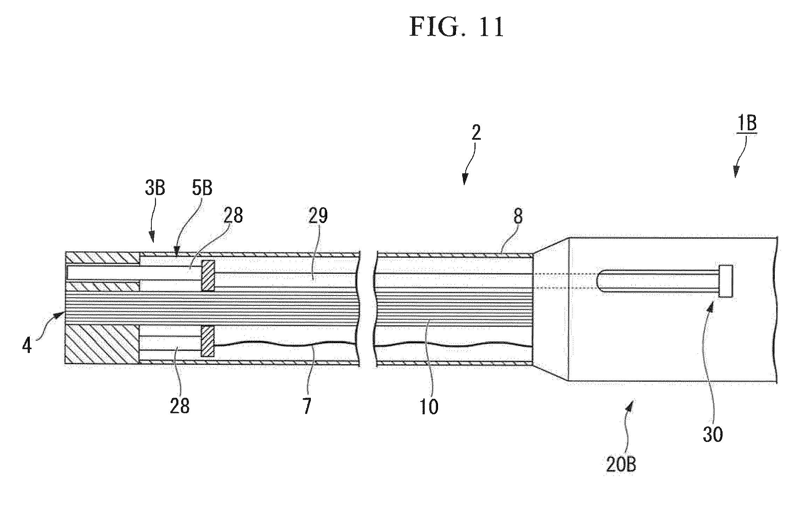

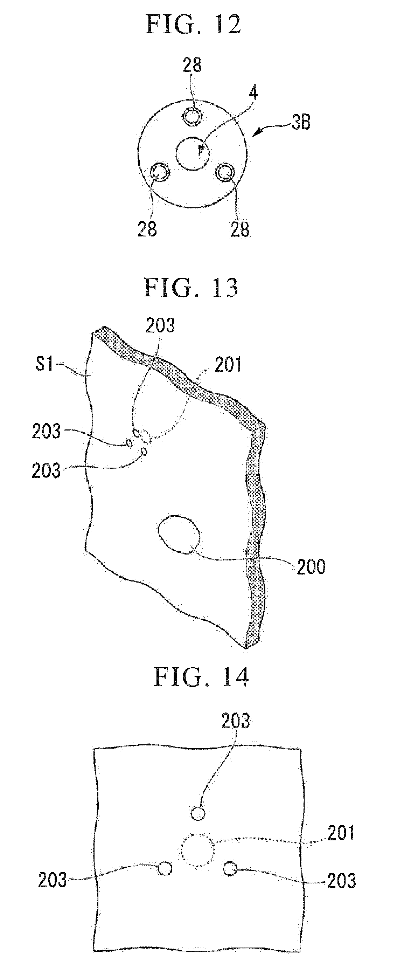

A medical device 1B according to a third embodiment of the present invention will be described. FIG. 11 is a partial sectional view of the medical device 1B according to the embodiment for illustrating the medical device 1B. FIG. 12 is a front view of the medical device 1B. FIG. 13 is a view illustrating a state after marking is performed using the medical device 1B. FIG. 14 is a view illustrating a positional relationship between markings formed by the medical device 1B.

As illustrated in FIGS. 11 and 12, the medical device 1B includes a distal configuration portion 3B provided with a plurality of bar-shaped electrodes 28 replacing the annular electrode 6, instead of the distal configuration portion 3 described in the first embodiment. The medical device 1B according to the embodiment includes a body portion 20B further having a slider 30 configured to operate the plurality of bar-shaped electrodes 28, instead of the body portion 20 described in the first embodiment.

The plurality of bar-shaped electrodes 28 are disposed in the distal portion of the insertion portion 2 so as to surround the light-emitting portion 4. A distal end of each of the bar-shaped electrodes 28 is a portion that is brought into contact with a biological tissue when a marking is formed using the bar-shaped electrode 28. The shape of the distal end of each of the bar-shaped electrodes 28 is not limited to a specific shape. For example, the shape of the distal end of each of the bar-shaped electrodes 28 may be a curved surface or a flat surface perpendicular to a longitudinal axis of the bar-shaped electrode 28 such that the distal end of the bar-shaped electrode 28 does not pierce a biological tissue. The wiring 7 for electrically connecting the bar-shaped electrodes 28 to the connector 26 (refer to FIG. 1) of the body portion 20B is fixed to a proximal end of each of the bar-shaped electrodes 28. A shaft 29 is attached to each of the bar-shaped electrodes 28. The shaft 29 connects the slider 30 and each of the bar-shaped electrodes 28 together and transmits the amount of operational force from the slider 30 to each of the bar-shaped electrodes 28. The shaft 29 is fixed to all of the bar-shaped electrodes 28. When the shaft 29 is moved in a longitudinal axial direction of the insertion portion 2, the bar-shaped electrodes 28 are moved integrally with the shaft 29 in the longitudinal axial direction of the insertion portion 2. For example, the shaft 29 may be a conductor, and instead of the wiring 7, the shaft 29 may be electrically connected to the connector 26.

The operating of the slider 30 of the body portion 20B causes the plurality of bar-shaped electrodes 28 to protrude from the distal end of the distal configuration portion 3 to the distal side, or causes the plurality of bar-shaped electrodes 28 to be accommodated in the distal configuration portion 3 such that a distal end surface of the distal configuration portion 3 is flush with the distal ends of the bar-shaped electrodes 28. In the embodiment, three bar-shaped electrodes 28 are provided in the distal end of the distal configuration portion 3B. The number of bar-shaped electrodes 28 may be one or two. In a case where three or more bar-shaped electrodes 28 surrounding the light-emitting portion 4 are provided in the distal configuration portion 3B, when markings are formed by the bar-shaped electrodes 28, the operator can easily recognize substantially the center of the markings as a marking position notified by the light-emitting portion 4 after the markings are formed. When four or more bar-shaped electrodes 28 are provided in the distal configuration portion 3B, for example, even if a marking formed by one of the bar-shaped electrodes 28 is unclear, the operator can easily recognize the position of substantially the center of markings.

In the embodiment, in the cross-sectional surface perpendicular to the longitudinal axis of the insertion portion 2, the light-emitting portion 4 is disposed in an inner region of an envelope of distal end portions of the plurality of bar-shaped electrodes 28 which are brought into contact with a biological tissue when markings are formed. That is, in the embodiment, a position of the light-emitting portion 4 and a position of the marking portion 5B have a fixed relationship with each other in the cross-sectional surface perpendicular to the longitudinal axis of the insertion portion 2, is fixed.

The configuration in which the plurality of bar-shaped electrodes 28 are used to form markings can allow an energy density when the plurality of bar-shaped electrodes 28 are energized with high-frequency current to be increased compared to the first embodiment in which the annular electrode 6 is used. For this reason, in the embodiment, the total amount of energy of high-frequency current required to form the markings can be reduced compared to the first embodiment.

As illustrated in FIGS. 13 and 14, in the embodiment, for example, when the operator forms markings on both the internal surface and the external surface of the stomach similar to the first embodiment, first, the operator forms markings 203 on the internal surface S2 of the stomach by moving the plurality of bar-shaped electrodes 28 to the distal side and energizing the bar-shaped electrodes 28 with high-frequency current. Subsequently, the operator moves the plurality of bar-shaped electrodes 28 to a proximal side, brings the distal end surface of the distal configuration portion 3B into contact with the internal surface S2 of the stomach, and thereafter, notifies the positions of the markings using light transmitted to the external surface S1 of the stomach. In the embodiment, the markings 203 formed on the internal surface S2 of the stomach is formed at positions that correspond to respective apexes of a triangular shape containing a site, the position of which is notified on the outside of the stomach by the light-emitting portion 4, inside the triangular shape. Accordingly, similar to the first embodiment, the operator can accurately form the markings 203 on the internal surface S2 of the stomach and the marking 201 on the external surface S1 of the stomach within a short period of time.

In the embodiment, the plurality of bar-shaped electrodes 28 are moved to the distal side, thereby allowing an increase in the distance between the light-emitting portion 4 and the distal ends of the bar-shaped electrodes 28 in contact with a tissue during formation of the markings 203. For this reason, even if the tissue generates heat when the bar-shaped electrodes 28 are energized with high-frequency current, the generated heat is unlikely to adversely affect the light guide 10 of the light-emitting portion 4.

### (Fourth Embodiment)

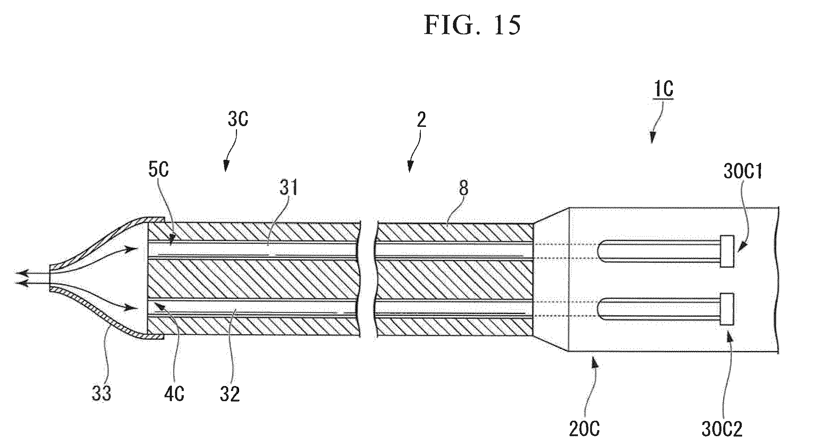

Next, a medical device 1C according to a fourth embodiment of the present invention will be described. FIG. 15 is a partial sectional view of the medical device 1C according to the embodiment for illustrating the medical device 1C.

As illustrated in FIG. 15, the medical device 1C according to the embodiment includes a distal configuration portion 3C including a movable electrode 31, a movable fiber 32, and a distal guide portion 33, instead of the distal configuration portion 3 described in the first embodiment. The medical device 1C according to the embodiment includes a body portion 20C further including a slider 30C1 and a slider 30C2 configured to move the movable electrode 31 and the movable fiber 32, instead of the body portion 20 described in the first embodiment.

The movable electrode 31 of the distal configuration portion 3C has a wire shape. A proximal portion of the movable electrode 31 is electrically connected to the connector 26 of the body portion 20C, and is fixed to the slider 30C1. Accordingly, in the embodiment, high-frequency current supplied from the high-frequency power supply device can be transmitted to the movable electrode 31, and markings can be formed using a distal end of the movable electrode 31.

The movable fiber 32 of the distal configuration portion 3C has a structure in which the light guide 10 described in the first embodiment can be advanced and retracted in the longitudinal direction of the insertion portion 2. That is, a proximal end of the movable fiber 32 is disposed in the body portion 20 such that light from the light emitter 23 can be incident to the movable fiber 32. The movable fiber 32 can be advanced and retracted in the longitudinal direction of the insertion portion 2 by the slider 30C2 of the body portion 20C. In the embodiment, the movable fiber 32 and the light emitter may be configured such that the movable fiber 32 and the light emitter can be integrally moved in the body portion 20C.

The distal guide portion 33 is a hollow member which includes one distal opening into which the movable electrode 31 and the movable fiber 32 can be inserted, and the diameter of which is gradually reduced from the proximal side toward the distal side.

In the embodiment, when the movable fiber 32 is exposed from the distal opening of the distal guide portion 33, a tissue can be illuminated with light emitted from the light emitter 23. That is, when the movable fiber 32 is disposed at the distal opening, a distal end of the movable fiber 32 becomes a light-emitting portion 4C, and performs the same function as that of the light-emitting portion 4 described in the first embodiment when being positioned at the distal opening of the distal guide portion 33. When the movable electrode 31 is exposed from the distal opening of the distal guide portion 33, the distal end of the movable electrode 31 becomes a marking portion 5C, and performs the same function as that of the marking portion 5 described in the first embodiment when being positioned at the distal opening of the distal guide portion 33. That is, in the embodiment, the distal guide portion 33 guides each of the distal end of the movable electrode 31 and the distal end of the movable fiber 32 to the distal opening of the distal guide portion 33, and thus a position of the light-emitting portion 4C and a position of the marking portion 5C have a fixed relationship with each other when each of the light-emitting portion 4C and the marking portion 5C is used.

Also, the same effects as in the first embodiment are obtained in this configuration.

### (Fifth Embodiment)

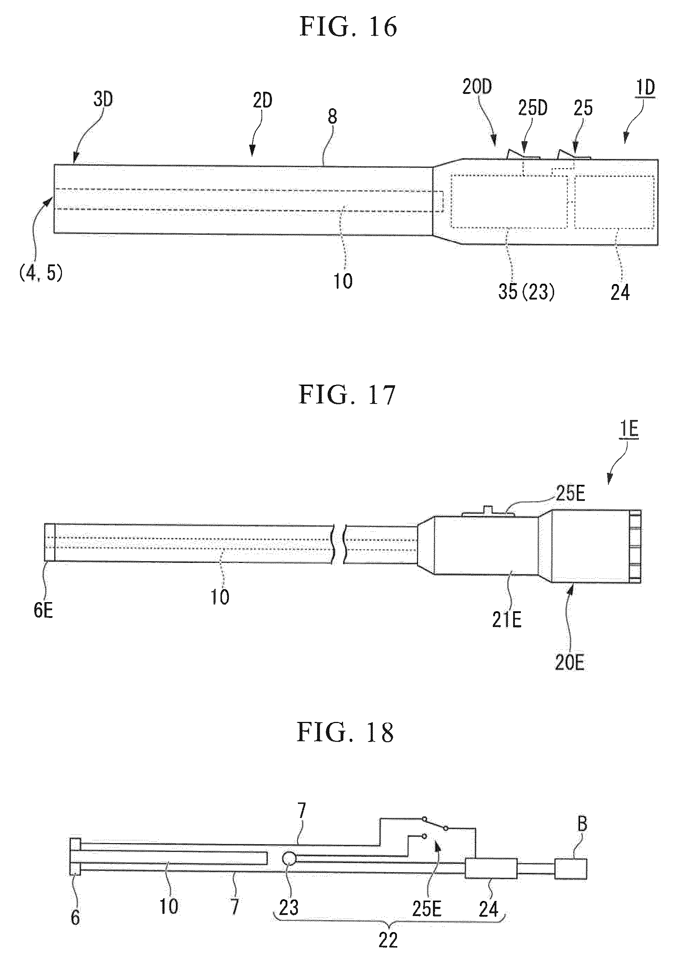

Next, a medical device 1D according to a fifth embodiment of the present invention will be described. FIG. 16 is an overall view illustrating the medical device 1D according to the embodiment.

As illustrated in FIG. 16, the medical device 1D is different from that in the first embodiment in that the marking portion 5 does not include the annular electrode 6 and performs marking using light transmitted through the light guide 10.

The medical device 1D includes an insertion portion 2D, a body portion 20D, and the light guide 10.

The insertion portion 2D includes a distal configuration portion 3D and the tubular portion 8.

The distal configuration portion 3D is a portion to which the distal end of the light guide 10 is fixed. In the embodiment, similar to the first embodiment, the distal configuration portion 3D includes the light-emitting portion 4 configured to emit light from the distal end of the light guide 10. In the embodiment, the light-emitting portion 4 also serves as the marking portion 5 performing marking using light transmitted through the light guide 10.

In the embodiment, high-energy laser light is used as light for marking. That is, in the embodiment, unlike the first embodiment, a laser light source 35 is adopted as the light emitter 23 in the body portion 20D.

In the embodiment, the laser light source 35 has a power output to the extent that a biological tissue can be cauterized. For example, the laser light source 35 is a semiconductor laser device, and emits laser light having a wavelength in a range from 790 nm to 830 nm. For example, the laser light source 35 has the maximum power output of 60 W.

In the embodiment, the laser light source 35 can change a power output in at least two stages. That is, the laser light source 35 can switch between a state in which the laser light source 35 emits laser light at the same power output of light emitted from a light-emitting diode described in the first embodiment and a state in which the laser light source 35 emits laser light having a power output required to form markings. As an example, the laser light source 35 in the embodiment includes a low-output light source and a high-output light source. The low-output light source emits laser light which is unlikely to heat a biological tissue and is easily seen through the biological tissue. The high-output light source emits laser light which easily heats a biological tissue. An example of the low-output light source is a light source emitting red laser light containing a small number of infrared components. An example of the high-output light source is a light source emitting red laser light or laser light having a wavelength greater than or equal to that of near-infrared light.

In addition to the configuration described in the first embodiment, the body portion 20D further includes a switch 25D for changing the power output of laser light. For example, laser light having a relatively low power output for transmitting marking positions from the internal surface S2 of the stomach to the external surface S1 of the stomach and laser light having a relatively high power output for forming markings on the internal surface S2 of the stomach are switched to each other by the operator's operation of the switch 25D.

As necessary, the power supply unit 24 and the switch 25D may be disposed outside the exterior body 21, and the power supply unit 24 and the laser light source 35 may be connected to each other by a cable or the like.

Unlike the first embodiment, in the embodiment, the electrode and the wiring 7 are not required, and it is not necessary to connect the medical device to the high-frequency power supply device. Since the power output of laser light is variable, light for the notification of marking positions and light for the formation of markings can be generated by the same light source. For this reason, the device configuration of the medical device 1D according to the embodiment is simplified.

In the embodiment, the distal end of the light guide 10 is the light-emitting portion 4 and also serves as the marking portion 5, and thus, a positional relationship between the light-emitting portion 4 and the marking portion 5 is always fixed.

The laser light source 35 may be configured to emit pulsed laser light for the notification of marking positions from the internal surface S2 of the stomach to the external surface S1 of the stomach, and to continuously emit laser light for the formation of markings on the internal surface S2 of the stomach. Accordingly, energy absorbed by a tissue can be differentiated.

When laser light is emitted to notify marking positions from the internal surface S2 of the stomach to the external surface S1 of the stomach, an ND filter may be interposed on an optical path so that energy absorbed by the tissue is decreased. Instead of the ND filter, a filter configured to cut off infrared region light may be interposed on the optical path.

### (Sixth Embodiment)

Next, a sixth embodiment of the present invention will be described. FIG. 17 is an overall view illustrating a medical device according to the embodiment. FIG. 18 is a schematic view illustrating the configuration of the medical device.

The configuration of a medical device 1E according to the embodiment is different from the configurations in the aforementioned embodiments in that markings are formed using a heat generator which includes an electrical resistor and is energized to generate heat.

Specifically, the medical device 1E includes a heat generator 6E instead of the annular electrode 6 described in the first embodiment. The medical device 1E does not include the connector 26 described in the first embodiment. Instead of the switch 25 described in the first embodiment, the medical device 1E includes a triple-pole switch 25E connected to the light emitter 23, the power supply unit 24, and the wiring 7.

The triple-pole switch 25E is fixed to an exterior body 21E which is provided in a body portion 20E having the same exterior shape as that of the body portion 20 described in the first embodiment. The triple-pole switch 25E is disposed such that the heat generator 6E is energized when the triple-pole switch 25E is moved to a proximal side (hand side) of the body portion 20E, and the light emitter 23 is energized when the triple-pole switch 25E is moved to a distal side of the body portion 20E.

The configuration of the heat generator 6E is not limited to a specific configuration. For example, the heat generator 6E includes a high-resistance conductive member such as a nichrome wire, and an insulator that coats the conductive member. The conductive member is connected to the wiring 7.

The same effects as in the aforementioned embodiments are obtained in the medical device 1E according to this embodiment.

The medical device 1E according to the embodiment does not require the plug cord that connects the high-frequency power supply device and the medical device 1 together in the first embodiment. As a result, a treatment can be performed in a cordless manner.

The preferred embodiments of the present invention have been described; however, the present invention is not limited to these embodiments and the modified example. Additions, omissions, replacements, other modifications can be made to the configurations without departing from the scope of the present invention.

The present invention is not limited to the aforementioned description, and is only limited by the scope of the appended claims.

### Industrial Applicability

According to the aforementioned embodiments (including the modified example), it is possible to accurately form markings on both of an internal surface and an external surface of a biological tissue within a short period of time.

### Reference Signs List

- 1A:: medical device
- 1B:: medical device
- 1C:: medical device
- 1D:: medical device
- 1E:: medical device
- 2:: insertion portion
- 4:: light-emitting portion
- 4C:: light-emitting portion
- 5:: marking portion
- 5B:: marking portion
- 5C:: marking portion
- 6:: annular electrode (electrode)

## Claims

1. A medical device comprising:
an insertion portion that includes a distal end and a proximal end and that is capable of being inserted into a body;
a light-emitting portion that is disposed at the distal end of the insertion portion and that is configured to emit light containing a wavelength capable of passing through a biological tissue; and
a marking portion that is disposed at the distal end of the insertion portion, that has a fixed positional relationship with the light-emitting portion, and that is configured to form a marking on the biological tissue.

2. The medical device according to Claim 1, wherein
the insertion portion includes a longitudinal axis that connects the distal end and the proximal end together, and
in a cross-sectional surface, which is perpendicular to the longitudinal axis, of the distal end of the insertion portion, the light-emitting portion is disposed in an inner region of a contour of the marking portion or in an inner region of an envelope of a portion of the marking portion which is capable of coming into contact with the biological tissue.

3. The medical device according to Claim 1, wherein the marking portion and the light-emitting portion are capable of being coaxially positioned.

4. The medical device according to Claim 1, wherein the marking portion includes an electrode capable of being energized with high-frequency current to cauterize the biological tissue.

5. The medical device according to Claim 1, wherein
the light-emitting portion is capable of emitting light, which has energy required to cauterize the biological tissue, to the biological tissue, and
a change in the energy allows the light-emitting portion to serve as the marking portion.
